# EUROPÄISCHE PATENTSCHRIFT

(11) **EP 0 349 934 B1**
(45) Veröffentlichungstag und Bekanntmachung des Hinweises auf die Patenterteilung: **11.06.1997**
(21) Anmeldenummer: 89112032.1
(22) Anmeldetag: 01.07.1989
(51) Int. Cl.: G01N 31/22, G01N 33/52, G01N 33/493, G01N 33/487

(54) **Verfahren zur Bestimmung der Ionenstärke oder des spezifischen Gewichts von wässrigen Flüssigkeiten**
Process for the determination of the ionic strength or the specific gravity of aqueous fluids
Procédé pour la détermination de la force ionique ou du poids spécifique des liquides aqueux

(30) Priorität: 08.07.1988 DE 3823151
(43) Veröffentlichungstag der Anmeldung: 10.01.1990
(73) Patentinhaber: BOEHRINGER MANNHEIM GMBH, 68298 Mannheim (DE)
(72) Erfinder: Mangold, Dieter, Dr.rer.nat., D-6701 Maxdorf (DE); Gambke, Brigitte, Dr.rer.nat., D-6800 Mannheim 1 (DE)

(56) Entgegenhaltungen:
- EP-A- 349 843
- US-A- 3 449 080
- US-A- 4 076 502
- CLINICAL CHEMISTRY, Band 28, Nr. 10, 1982, Seiten 2068-2072; A.E. BURKHARDT et al.: "A reagent strip for measuring the specific gravity of urine"

## Beschreibung

Gegenstand der Erfindung ist ein neues Verfahren zur Bestimmung der Ionenstärke oder des spezifischen Gewichts von wäßrigen Flüssigkeiten und ein Reagenz zu dessen Ausführung.

Zur Bestimmung des spezifischen Gewichts in Harn sind mehrere physikalische Methoden bekannt, beispielsweise die Bestimmung mit Hydrometern, Urinometern, Pyknometern und Refraktometern. Diese Methoden sind zwar von ausreichender Genauigkeit, erfordern aber einen hohen instrumentellen Aufwand und sind durch die erforderliche Reinigung und Kalibrierung der Geräte zeit- und kostenintensiv.

Einfacher ist die Messung des spezifischen Gewichts mit Hilfe sogenannter Teststreifen, die auch visuell ausgewertet werden können. Die Teststreifen weisen ein Testfeld auf, welches geeignete Reagenzien enthält. Die Bestimmung kann in einfacher Weise durch Eintauchen des Teststreifens in eine Harnprobe und Beobachtung der Verfärbung des Testfeldes geschehen. Ein Teststreifen bietet zudem den Vorteil der Kombinierbarkeit mit anderen Harntests, beispielsweise zum Nachweis von Glukose, Leukozyten, Blut etc., in einem Mehrfachteststreifen mit entsprechend vielen voneinander getrennten Testfeldern. Die Messung des spezifischen Gewichts von Harn kann dann zeitsparend in einem Schritt neben der Messung dieser Parameter erfolgen. Die Auswertung kann auch apparativ durchgeführt werden.

Ein Verfahren zur Bestimmung des spezifischen Gewichts von Harn auf einem Teststreifen ist in der US-A-4,015,462 beschrieben. Eine Trägermatrix ist mit osmotisch zerbrechlichen Mikrokapseln versehen, die eine Flüssigkeit enthalten; wird eine solche Matrix mit einer Probenlösung niedrigerer Osmolalität in Berührung gebracht, so steigt der hydrostatische Druck in den Mikrokapseln, deren Wandung aus halbdurchlässigem Membranmaterial besteht, an. Dies führt zu einem Platzen der Mikrokapseln. Das Platzen der Mikrokapseln und die damit einhergehende Freisetzung einer in der Flüssigkeit gelösten Farbe führt zur Verfärbung der Matrix. Die Intensität der Färbung ist proportional zur Osmolalität bzw. zum spezifischen Gewicht der Probelösung. Ein Nachteil dieses Verfahrens ist die Verwendung der mit Mikrokapseln versehenen Trägermatrix, da ihre Herstellung wegen der erforderlichen Genauigkeit sehr schwierig ist.

Ein weiteres Verfahren zur Bestimmung des spezifischen Gewichts oder der Ionenstärke auf einem Teststreifen wird in der US-A-4,318,709 und der korrespondierenden DE-A 29 44 980 beschrieben. Darin wird die Probe mit einem Reagenz versetzt, welches ein schwach saueres oder schwach basisches, zu wenigstens 50 % neutralisiertes Polyelektrolytpolymer und einen Indikator enthält. Das zugrundeliegende Prinzip ist die Proportionalität zwischen dem spezifischen Gewicht und der Ionenstärke einer wäßrigen Lösung.

Als Polyelektrolytpolymere werden dort Polymere mit ionischen Gruppen bezeichnet. Beispielhaft genannt sind Polyacrylsäure oder Polyvinylamin.

In der EP-A-0 114 315 wird ein Reagenz beschrieben, das ein schwach basisches, durch eine starke organische Säure neutralisiertes Polyelektrolytpolymer und einen Indikator enthält.

In der EP-A-0 114 316 wird ein Reagenz offenbart, das ein schwach saueres Polyelektrolytpolymer, in welchem mindestens eine Carboxylgruppe in Form eines Ammoniumsalzes vorliegt, und einen Indikator enthält.

Auch in der EP-A-0 023 631 wird ein Reagenz zur Bestimmung der Ionenstärke oder des spezifischen Gewichts beschrieben. Es enthält neben einem stark saueren oder stark basischen Polyelektrolytpolymer eine Puffersubstanz, die einen pH-Wert von mindestens 6.5 gewährleistet, sowie einen pH-Indikator.

Verfahren, die mit einem Reagenz durchgeführt werden, welches Polyelektrolytpolymere enthält, haben jedoch den Nachteil, daß die durch den pH-Sprung einmal hervorgerufene Farbe sich mit der Zeit verändert. Das hat zur Folge, daß sich der Wert, den man für das spezifische Gewicht nach einer Minute abliest, sich von dem Wert, wenn man nach zwei oder gar fünf Minuten abliest, stark unterscheidet.

Wenn der Test also von nicht speziell dafür ausgebildeten Personen, z. B. beliebigen Patienten, ausgeführt wird, und die Auswertung nicht immer zur gleichen, vom Hersteller des Streifens vorgegebenen Zeit vorgenommen wird, kann das zu folgenschweren Fehlinterpretationen des Gesundheitszustandes führen.

Es bestand daher ein Bedarf an einem Verfahren zur Bestimmung der Ionenstärke oder des spezifischen Gewichts, welches Versuchsergebnisse liefert, die während des Tests über längere Zeit hinweg konstant bleiben.

Es war Aufgabe der vorliegenden Erfindung, diesen Bedarf zu befriedigen und Verfahren zur Verfügung zu stellen, die auch auf Testträgern schnell und zuverlässig sind.

Die Aufgabe wird gelöst von dem erfindungsgemäßen Verfahren zur Bestimmung der Ionenstärke oder des spezifischen Gewichts einer wäßrigen Flüssigkeit durch Vermischen der Flüssigkeit mit einem geeigneten Reagenz, Bestimmung des pH-Sprunges in der resultierenden Flüssigkeit und Auswertung, dadurch gekennzeichnet, daß das Reagenz mindestens eine pH-Puffersubstanz,jedoch kein Polyelektrolytpolymer,oder mindestens eine pH-Puffersubstanz und/oder mindestens einen Komplexbildner enthält.

Ein weiterer Gegenstand der Erfindung ist ein dafür geeignetes Reagenz.

Die wäßrige Flüssigkeit, deren Ionenstärke, auch Osmolalität genannt, oder deren spezifisches Gewicht in dem erfindungsgemäßen Verfahren ermittelt werden kann, besteht im wesentlichen aus Wasser. Weitere Bestandteile der Flüssigkeit können gelöste oder/und nicht gelöste Bestandteile sein. Gelöste Bestandteile sind ionische und nichtionische Substanzen. Ungelöste Bestandteile können schwerlösliche chemische Substanzen, aber auch andere Materialien, wie biologische Substanzen, beispielsweise Zellen, sein.

Die Flüssigkeit kann einen beliebigen pH-Wert aufweisen. Bevorzugt können Flüssigkeiten mit einem pH-Wert von 3 bis 13, bevorzugt von 4 bis 9 untersucht werden. Es können auch Flüssigkeiten mit einem anderen pH-Wert untersucht werden, wenn deren pH-Wert durch Zugabe von Säuren oder Basen, vor oder während der Bestimmung in diesen Bereich gebracht wird.

Falls das Verfahren zur Bestimmung des spezifischen Gewichts benutzt wird, kann es für einen Bereich der Werte des spezifischen Gewichts von 0.95 bis 2, bevorzugt von 0.99 bis 1.2, eingesetzt werden.

Bevorzugte wäßrige Flüssigkeiten sind Salzlösungen und Körperflüssigkeiten. Das erfindungsgemäße Verfahren ist besonders gut für Körperflüssigkeiten, wie Schweiß und Harn, geeignet. Insbesondere hat es sich zur Harnuntersuchung als geeignet erwiesen. In diesem Fall kann nämlich eine Probe des Harns ohne weitere Vorbereitungsschritte mit dem Reagenz versetzt werden, da sowohl das spezifische Gewicht des Harns mit Werten von 1.00 bis 1.04 als auch der pH-Wert des Harns mit Werten von 4,5 bis 8,0 normalerweise in dem für das erfindungsgemäße Verfahren bevorzugten Bereich liegen.

Das Reagenz, mit welchem die Flüssigkeit vermischt wird, enthält mindestens eine pH-Puffer-Substanz oder/und mindestens einen Komplexbildner. pH-Puffer-Substanzen sind dem Fachmann bekannte Substanzen. pH-Puffer-Substanz ist eine Mischung einer schwachen Säure mit einem praktisch vollständig dissoziierten Salz dieser Säure bzw. eine Mischung einer schwachen Base mit einem praktisch vollständig dissoziierten Salz dieser Base. Dies geht beispielsweise aus Römpps Chemie Lexikon, 8. Auflage, Band 5, Stichwort "Puffer", hervor. Dort wird auch die Wirkung einer Lösung dieser Puffersubstanzen gegenüber zugegebenen Säuren und Basen beschrieben; der pH-Wert des Puffers ändert sich bei Zugabe von Säuren oder Basen kaum.

Gemäß der vorliegenden Erfindung sind als Reagenz für den Einsatz bei der Bestimmung der Ionenstärke bzw. des spezifischen Gewichts, insbesondere von Harn, pH-Puffer-Substanzen geeignet, deren pK-Wert in Wasser zwischen 4 und 12, bevorzugt zwischen 5,5 und 10 liegt. Von diesen sind Puffersubstanzen mit einem möglichst kleinen Aktivitätskoeffizienten bevorzugt. Der Aktivitätskoeffizient ist besonders klein bei Puffersubstanzen, die aus Ionen mit hoher effektiver Ladung aufgebaut sind. Die Aktivitätskoeffizienten von Ionen und Puffersubstanzen in wäßrigen Lösungen sind bekannt oder können auf bekannte Weise einfach bestimmt werden (F. Seel, Grundlagen der analytischen Chemie, Verlag Chemie, Weinheim; Broschüre "Buffers" der Firma Calbiochem., 1985, Seiten 12 -15). Als besonders bevorzugt haben sich Puffersubstanzen erwiesen, deren Aktivitätskoeffizienten bei einer Konzentration von 0,1 M kleiner als 1 und bevorzugt kleiner als 0,9 ist.

Solche Puffersubstanzen sind in einem pH-Bereich von 4 bis 11 beispielsweise Puffer, die folgende Ionen enthalten: Phosphat, Borat, Carbonat, Citrat, Diäthylmalonat, Nitrilo-tris-methylenphosphonat. Ebenfalls geeignet sind zwitterionische Puffer, wie beispielsweise Glycinpuffer oder 2(N-Morpholino)äthansulfonsaure (MES).

Komplexbildner im Sinne der Erfindung sind chemische Substanzen, welche mit Ionen unter Komplexbildung reagieren. Besonders bevorzugt sind Komplexbildner, bei deren Komplexbildung mit Ionen mindestens ein Proton freigesetzt wird.

Solche Komplexbildner sind beispielsweise Kronenether, Kryptanden, Podanden und mehrzähnige Liganden, die schwach saure oder schwach basische Gruppen, wie beispielsweise Carboxyl- oder Aminogruppen besitzen. Bevorzugt sind Komplexbildner, die maximal 1 Ion pro Molekül komplexieren können. Zur besonders genauen Erfassung der Ionenstärke bzw. des spezifischen Gewichts muß der Komplexbildner in der Lage sein, mit möglichst allen in der Flüssigkeit vorhandenen Ionen, insbesondere den beispielsweise in Harn oft vorkommenden Kationen, wie Alkali- und Erdalkaliionen Komplexe bilden zu können. Für den Fall, daß in dem erfindungsgemäßen Verfahren ein pH-Indikator zur Messung des pH-Wertes eingesetzt wird, ist die Verwendung von farblosen Komplexbildnern bevorzugt. Daher sind als Komplexbildner beispielsweise folgende Verbindungen geeignet:
- Kronenether, Kryptanden, Podanden:
   18-Krone-6-tetracarbonsäure, N-Phenylaza-15-Krone-5, Hexacylentrisulfat, Kryptofix 222^{R}, Kryptofix 221^{R}, Kryptofix 211^{R},
- mehrzähnige Liganden:
   Ethylendiamintetraessigsäure, Nitrilotriessigsäure, Diethylentriaminpentaessigsäure, Di-(2-aminoethoxy)-ethantetraessigsäure, Hexamethylendinitrilotetraessigsäure, Nitrilodiessigsäure, N-Methylaminodiessigsäure.

Für Bestimmungen der Ionenstärke oder des spezifischen Gewichts von Flüssigkeiten, welche hauptsächlich Salze gelöst enthalten, die keine wesentliche Pufferkapazität aufweisen, führt man das Verfahren besonders bevorzugt mit einem Reagenz aus, das hauptsächlich aus einem oder mehreren Komplexbildnern besteht. In diesem Fall muß das Reagenz nicht unbedingt pH-Puffersubstanzen enthalten.

Soll das spezifische Gewicht einer Flüssigkeit bestimmt werden, die Substanzen mit merklicher Pufferkapazität, beispielsweise schwache Basen oder Säuren bzw. ihre Salze, beinhalten, so ist ein Reagenz bevorzugt, welches mindestens eine pH-Puffersubstanz enthält.In In diesem Fall wird auch der Beitrag nichtionischer Verbindungen, die in der Flüssigkeit enthalten sein können, zum spezifischen Gewicht berücksichtigt. Zusätzlich kann das Reagenz dann auch einen oder mehrere Komplexbildner enthalten. Für die Untersuchung von Harn sollte eine Reagenz-Zusammensetzung gewählt werden, die, aufgelöst in einer zur Probe äquivalenten Menge Wasser, einen pH-Wert von vorzugsweise größer als 5,5 aufweist.

Aus Gründen der Einfachheit ist es zweckmäßig, die Zahl der Komponenten möglichst klein zu halten. Daher ist es auch sehr vorteilhaft, als Puffersubstanz eine Substanz zu wählen, die über ihre pH-puffernde Wirkung hinaus auch ionen-komplexierende Wirkung hat. Solche Substanzen sind beispielsweise Nitrilo-tris(methylenphosphonsäure) und Pentanatriumtriphosphat (Na₅P₃O₁₀).

Die Zusammensetzung des Reagenzes, insbesondere bezüglich seines pH-Wertes, wird bevorzugt so gewählt, daß der pH-Wert nach Auflösung des Reagenzes in einer zu der Probe gleichen Menge an Wasser ungefähr gleich oder größer und besonders bevorzugt größer ist als der pH-Wert der zu untersuchenden Flüssigkeit. Der am besten geeignete pH-Wert kann von einem Fachmann ohne weiteres anhand weniger Versuche ermittelt werden.

Im Falle eines auf einen saugfähigen Träger aufgebrachten Reagenzes ist dies sehr einfach zu bewerkstelligen. Der pH-Wert der Tränklösung wird dann vor der Imprägnierung entsprechend eingestellt, beispielsweise bei einem Reagenz zur Bestimmung des spezifischen Gewichts von Harn auf einen pH-Wert von 6 bis 11, bevorzugt von 7 bis 9.

Die Menge an pH-Puffersubstanzen oder/und Komplexbildnern im Reagenz, bezogen auf die Menge an damit gemischter zu untersuchender Flüssigkeit wird so gewählt, daß die Endkonzentration dieser Substanzen an Reagenz in der Probenflüssigkeit insgesamt 0,005 bis 1,0 mol/l, bevorzugt 0,005 bis 0,2 beträgt. Die Form, in der das Reagenz vorliegt, ist für die Bestimmung nicht von wesentlicher Bedeutung. Beispielsweise kann das Reagenz als Pulver, Tablette oder in Form einer Lösung verwendet werden.

Bei der bevorzugten Ausführungsform des Verfahrens auf einem Teststreifen ist das Reagenz zweckmäßig als Überzug auf einen saugfähigen Träger aufgebracht. Bevorzugte Träger sind poröse Trägermaterialien oder Vliese. Als Vliese werden papierartig aus Fasern aufgebaute Träger verstanden. Mit Reagenz imprägnierte saugfähige Träger werden bevorzugt hergestellt, indem man das saugfähige Trägermaterial mit einer Lösung des Reagenzes tränkt und trocknet. Dadurch bildet sich auf der ganzen, bevorzugt auch der inneren, Oberfläche ein Reagenzfilm.

Das Reagenz kann auch mit filmbildenden und quellfähigen Zusatzstoffen auf einen Träger, beispielsweise direkt auf dem Teststreifen, aufgebracht sein. Das Reagenz kann weiterhin übliche Zusatzstoffe, wie beispielsweise Stabilisatoren, Netzmittel oder Quellmittel enthalten.

Das Vermischen der Flüssigkeit mit dem Reagenz geschieht in einer Art und Weise, wie sie für die für das Reagenz gewählte Form zweckmäßig ist. Wenn das Reagenz als Überzug auf einem saugfähigen Träger aufgebracht ist, dann kann einfach der Träger mit der Flüssigkeit in Kontakt gebracht werden. Die Flüssigkeit verteilt sich dann im Träger und löst die Reagenzien ab. Dieser Lösevorgang geht mit dem Reagenz des erfindungsgemäßen Verfahrens besonders schnell und vollständig vor sich, da es sich bei den Reagenzien um leichtlösliche niedermolekulare Substanzen handelt.

Hervorgerufen durch die Einwirkung des Reagenzes auf die zu untersuchende Flüssigkeit stellt sich ein von der Ionenstärke oder dem spezifischen Gewicht der zu untersuchenden Flüssigkeit abhängiger pH-Wert ein.

Im erfindungsgemäßen Verfahren wird dieser pH-Wert bestimmt. Das kann auf bekannte Art, beispielsweise auch mit einer pH-Elektrode, geschehen. Besonders zweckmäßig ist die Bestimmung mit Hilfe eines pH-Indikators, da die Bestimmung dann auch visuell und ohne zusätzliche Geräte durchgeführt werden kann.

Voraussetzung ist, daß ein pH-Indikator benutzt wird, der einen Farbumschlag in einem pH-Bereich, der den sich in der Flüssigkeit einstellenden pH umfaßt, aufweist. Solche pH-Indikatoren und ihr Farbumschlagsbereich sind bekannt. Für die Bestimmung der Ionenstärke oder des spezifischen Gewichts von Harn sind pH-Indikatoren mit einem pK-Wert von 4 bis 12 geeignet. Als besonders gut geeignet haben sich davon Bromthymolblau oder Thymolblau erwiesen.

Dieser pH-Indikator kann dem Reagenz oder der zu untersuchenden Flüssigkeit schon vor dem Vermischen zugesetzt sein, oder er kann nach dem Vermischen oder während des Vermischens des Reagenzes mit der Flüssigkeit zugegeben werden. Die Menge des zugesetzten Indikators wird so bemessen, daß der Farbumschlag deutlich bestimmbar ist. Bevorzugte Konzentrationen des Indikators in der Mischung aus Reagenz und Probenflüssigkeit sind 0,1 bis 100 mmol/l, bevorzugt 1 bis 50 mmol/l.

Zwar ist es in dem erfindungsgemäßen Verfahren möglich, daß die Reaktion des Reagenzes mit der wäßrigen Flüssigkeit in einem Zweiphasensystem, beispielsweise in zwei nicht oder nur wenig ineinander löslichen flüssigen Phasen, durchgeführt wird, wobei sich mindestens ein Teil des Reagenzes in der nicht wäßrigen Phase befindet; jedoch ist der Fall bevorzugt, bei dem während der Reaktion des Reagenzes mit der Flüssigkeit bzw. ihrer Bestandteile alle Reagenzien, insbesondere die pH-Puffersubstanzen bzw. Komplexbildner und gegebenenfalls der pH-Indikator, in einer Phase, bevorzugt in der wäßrigen Phase, gelöst sind. In diesem Fall verläuft die Reaktion besonders schnell.

Die Auswertung im erfindungsgemäßen Verfahren kann auf besonders einfache Art geschehen. Beispielsweise kann dem ermittelten pH-Wert die zugehörige Ionenstärke oder das spezifische Gewicht mittels einer Eichkurve zugeordnet werden. Die Eichkurve erhält man, indem man die pH-Werte für vergleichbare Lösungen, die eine jeweils verschiedene, aber bekannte Ionenstärke bzw. ein verschiedenes, aber bekanntes spezifisches Gewicht haben, bestimmt. Im Falle der visuellen pH-Bestimmung mit Hilfe eines pH-Indikators kann die entstandene Farbe beispielsweise auch mit Farben verglichen werden, die für eine Reihe von Flüssigkeiten bekannter Ionenstärke oder spezifischen Gewichts erhalten und festgehalten wurden, zum Beispiel in einer Farbskala.

Die Auswertung kann schon nach weniger als einer Minute nach Vermischen von Reagenz und wäßriger Probenflüssigkeit vorgenommen werden. Das erfindungsgemäße Verfahren hat den Vorteil, daß der ermittelte pH-Wert auch über 5 Minuten nach dem Vermischen immer noch konstant ist. Dies ist insbesondere dann wichtig, wenn die Auswertung längere Zeit in Anspruch nimmt oder die Auswertung kurze Zeit nach der ersten Auswertung zur Sicherheit wiederholt werden soll.

Eine besonders bevorzugte Ausführungsform des erfindungsgemäßen Verfahrens ist ein Verfahren zur Bestimmung des spezifischen Gewichts von Harn auf einem Teststreifen. Dieser Teststreifen weist ein Testfeld auf, welches aus einem mit einer Lösung des Reagenz imprägnierten und getrockneten Vlies besteht. Als Reagenz findet ein Gemisch aus einer pH-Puffersubstanz, beispielsweise einem Phosphatpuffer, der bei Auflösen in Wasser einen pH-Wert von 8 aufweist, einem Komplexbildner, beispielsweise Nitrilotriessigsäure, und einem pH-Indikator, wie Bromthymolblau, Verwendung. Das Testfeld wird mit dem zu untersuchenden Harn in Kontakt gebracht, beispielsweise durch Eintauchen des Teststreifens in den Harn und Herausnahme, sobald das Testfeld naß ist. Die Farbe, die das Testfeld dann angenommen hat, wird mit der Farbe eines dem Teststreifen beiliegenden Musters verglichen. Jeder Farbe des Musters ist dabei ein Wert für ein spezifisches Gewicht zugeordnet. Das zu bestimmende spezifische Gewicht hat den Wert, der dem Muster mit übereinstimmender Farbe zugeordnet ist.

Die Erfindung wird durch die folgenden Beispiele weiter erläutert:

### Beispiel 1

a) Herstellung eines Teststreifens zur Bestimmung der Ionenstärke oder des spezifischen Gewichts von Salzlösungen oder Harn
Ein Cellulosevlies (Typ 23 SL, Firma Schleicher & Schüll) wurde mit einer wäßrigen Lösung folgender Zusammensetzung getränkt:

| | |
|---|---|
| Nitrilo-tris(methylenphosphonsäure): | 120 mM |
| Bromthymolblau: | 0,15 Gew.-% |

In der Lösung war mit 3.5 N wäßriger Tetramethylammoniumhydroxid-Lösung ein pH-Wert von 8 eingestellt worden.
Das Vlies wurde getrocknet und auf die Größe von 5 ∗ 5 mm geschnitten. Das so hergestellte Testfeld wurde auf einen Kunststoffstreifen (100 ∗ 5 mm) aufgeklebt.
b) Ein Cellulosevlies (Typ 23 SL, Firma Schleicher & Schüll) wurde mit einer wäßrigen Lösung folgender Zusammensetzung getränkt:

| | |
|---|---|
| Pentanatriumtriphosphat (Na₅P₃O₁₀) | 60 mM |
| Bromthymolblau | 0,15 Gew.-% |

In der Lösung war mit 2 N Salzsäure ein pH-Wert von 8 eingestellt worden.
Das Vlies wurde getrocknet und auf die Größe von 5 ∗ 5 mm geschnitten. Das so hergestellte Testfeld wurde auf einen Kunststoffstreifen (100 ∗ 5 mm) aufgeklebt.
c) Ein Cellulosevlies (Typ 2316, Firma Binzer) wurde mit einer wäßrigen Lösung folgender Zusammensetzung getränkt:

| | |
|---|---|
| Ethylendiamintetraessigsäure | 0.2 Gew.-% |
| Natriumtetraborat | 60 mM |
| Thymolblau | 0,1 Gew.-% |

In der Lösung war mit 10 N Natronlauge ein pH-Wert von 10 eingestellt worden.
Das Vlies wurde getrocknet und auf die Größe von 5 ∗ 5 mm geschnitten. Das so hergestellte Testfeld wurde auf einen Kunststoffstreifen (100 ∗ 5 mm) aufgeklebt.
d) Ein Cellulosevlies (Typ 23 SL, Firma Schleicher & Schüll) wurde mit einer wäßrigen Lösung folgender Zusammensetzung getränkt:

| | |
|---|---|
| Natriumdihydrogenphosphat | 60 mM |
| 2-(N-Morpholino)-äthansulfonsäure (MES) | 40 mM |
| Bis-(aminoethyl)-glycoläther-N,N,N',N'-tetraessigsäure | 0.75 Gew.-% |
| Bromthymolblau | 0,15 Gew.-% |

In der Lösung war mit 10 N Natronlauge ein pH-Wert von 8 eingestellt worden.
Das Vlies wurde getrocknet und auf die Größe von 5 ∗ 5 mm geschnitten. Das so hergestellte Testfeld wurde auf einen Kunststoffstreifen (100 ∗ 5 mm) aufgeklebt.
e) Ein Cellulosevlies (Typ 2316, Firma Binzer) wurde mit einer wäßrigen Lösung folgender Zusammensetzung getränkt:

| | |
|---|---|
| Natriumdihydrogenphosphat | 30 mM |
| Nitrilotriessigsäure | 0.2 Gew.-% |
| Bromthymolblau | 0,1 Gew.-% |

In der Lösung war mit 10 N Natronlauge ein pH-Wert von 8 eingestellt worden.
Das Vlies wurde getrocknet und auf die Größe von 5 ∗ 5 mm geschnitten. Das so hergestellte Testfeld wurde auf einen Kunststoffstreifen (100 ∗ 5 mm) aufgeklebt.
f) Ein Cellulosevlies (Typ 23 SL, Firma Schleicher & Schüll) wurde mit einer wäßrigen Lösung folgender Zusammensetzung getränkt:

| | |
|---|---|
| Ethylendiamintetraessigsäure | 0.2 Gew.-% |
| Natriumdihydrogenphosphat | 30 mM |
| Bromthymolblau | 0,1 Gew.-% |

In der Lösung war mit 10 N Natronlauge ein pH-Wert von 8 eingestellt worden.
Das Vlies wurde getrocknet und auf die Größe von 5 ∗ 5 mm geschnitten. Das so hergestellte Testfeld wurde auf einen Kunststoffstreifen (100 ∗ 5 mm) aufgeklebt.
g) Ein Cellulosevlies (Typ 23 SL, Firma Schleicher & Schüll) wurde mit einer wäßrigen Lösung folgender Zusammensetzung getränkt:

| | |
|---|---|
| Diäthylmalonsäure | 50 mM |
| Nitrilotriessigsäure | 0.5 Gew.-% |
| Bromthymolblau | 0,15 Gew.-% |

In der Lösung war mit 3,5 N Tetramethylammoniumhydroxidlösung ein pH-Wert von 8 eingestellt worden.

Das Vlies wurde getrocknet und auf die Größe von 5 ∗ 5 mm geschnitten. Das so hergestellte Testfeld wurde auf einen Kunststoffstreifen (100 ∗ 5 mm) aufgeklebt.

### Beispiel 2

### Bestimmung des spezifischen Gewichts einer Harnprobe

Ein nach Beispiel 1 hergestellter Teststreifen wurde kurz in Harn eingetaucht. Nach 30 Sekunden, 1 Minute, 2 Minuten bzw. 5 Minuten wurde die Farbe mit den Farben einer Farbskala verglichen, die für Harne bekannten spezifischen Gewichts erhalten wurden. Die zu den oben angegebenen Meßzeiten für das spezifische Gewicht erhaltenen Werte waren gleich. Das spezifische Gewicht stimmte mit dem mittels eines Pyknometers ermittelten spezifischen Gewichts dieses Harns überein.

## Patentansprüche

1. Verfahren zur Bestimmung der Ionenstärke oder des spezifischen Gewichts einer wäßrigen Flüssigkeit durch Vermischen der Flüssigkeit mit einem geeigneten Reagenz, Bestimmung des pH-Wertes in der Flüssigkeit und Auswertung, dadurch gekennzeichnet, daß das Reagenz einen pH-Indikator sowie mindestens
- eine pH-Puffersubstanz, jedoch kein Polyelektrolytpolymer, oder
- mindestens eine pH-Puffersubstanz und/oder
- mindestens einen Komplexbildner enthält.

2. Verfahren gemäß Anspruch 1, dadurch gekennzeichnet, daß das Reagenz auf einen saugfähigen Träger imprägniert ist.

3. Verfahren gemäß einem der Ansprüche 1 oder 2, dadurch gekennzeichnet, daß die pH-Puffersubstanz bei einer Konzentration von 0,1 M einen Aktivitätskoeffizienten von kleiner als 1 aufweist.

4. Reagenz zur Bestimmung der Ionenstärke oder des spezifischen Gewichts einer wäßrigen Flüssigkeit, dadurch gekennzeichnet, daß es einen pH-Indikator sowie
- eine pH-Puffersubstanz, jedoch kein Polyelektrolytpolymer, oder
- mindestens eine pH-Puffersubstanz und/oder
- mindestens einen Komplexbildner enthält.

5. Reagenz gemäß Anspruch 4, dadurch gekennzeichnet, daß es auf einen saugfähigen Träger imprägniert ist.

6. Reagenz gemäß einem der Ansprüche 4 oder 5, dadurch gekennzeichnet, daß die pH-Puffersubstanz bei einer Konzentration von 0,1 M einen Aktivitätskoeffizienten von kleiner als 1 aufweist.

7. Teststreifen zur Bestimmung der Ionenstärke oder des spezifischen Gewichts einer wäßrigen Flüssigkeit, bestehend aus einem Kunststoffstreifen und einem darauf befestigten, mit dem Reagenz imprägnierten saugfähigen Träger, dadurch gekennzeichnet, daß als Reagenz das Reagenz des Anspruchs 4 dient.

## Claims

1. Process for the determination of the ionic strength or of the specific weight of an aqueous liquid by mixing of the liquid with a suitable reagent, determination of the pH value in the liquid and evaluation, characterised in that the reagent contains a pH indicator, as well as at least
- one pH buffer substance but no polyelectrolyte polymer, or
- at least one pH buffer substance and/or
- at least one complex former.

2. Process according to claim 1, characterised in that the reagent is impregnated on to an absorbent carrier.

3. Process according to one of claims 1 or 2, characterised in that the pH buffer substance has an activity coefficient of less than 1 at a concentration of 0.1 M.

4. Reagent for the determination of the ionic strength or of the specific weight of an aqueous liquid, characterised in that it contains a pH indicator, as well as
- a pH buffer substance but no polyelectrolyte polymer, or
- at least one pH buffer substance and/or
- at least one complex former.

5. Reagent according to claim 4, characterised in that it is impregnated on to an absorbent carrier.

6. Reagent according to one of claims 4 or 5, characterised in that the pH buffer substance has an activity coefficient of less than 1 at a concentration of 0.1 M.

7. Test strip for the determination of the ionic strength or of the specific weight of an aqueous liquid, consisting of a synthetic resin strip and an absorbent carrier impregnated with the reagent fixed thereon, characterised in that the reagent of claim 4 serves as reagent.

## Revendications

1. Procédé de détermination de la force ionique ou du poids spécifique d'un liquide aqueux, consistant à mélanger le liquide avec un réactif approprié, à déterminer le pH du liquide et à effectuer une évaluation, caractérisé en ce que le réactif contient un indicateur de pH et au moins
- une substance tampon de pH, mais pas de polymère électrolytique, ou
- au moins une substance tampon de pH et/ou
- au moins un agent complexant.

2. Procédé selon la revendication 1, caractérisé en ce que le réactif imprègne un support absorbant.

3. Procédé selon l'une quelconque des revendications 1 ou 2, caractérisé en ce que la substance tampon de pH présente, à une concentration de 0,1 M, un coefficient d'activité inférieur à 1.

4. Réactif destiné à la détermination de la force ionique ou du poids spécifique d'un liquide aqueux, caractérisé en ce qu'il contient un indicateur de pH et
- une substance tampon de pH, mais pas de polymère électrolytique, ou
- au moins une substance tampon de pH et/ou
- au moins un agent complexant.

5. Réactif selon la revendication 4, caractérisé en ce que le réactif imprègne un support absorbant.

6. Réactif selon l'une quelconque des revendications 4 ou 5, caractérisé en ce que la substance tampon de pH présente, à une concentration de 0,1 M, un coefficient d'activité inférieur à 1.

7. Bandes de test destinées à la détermination de la force ionique ou du poids spécifique d'un liquide aqueux, constituées d'une bande en matière plastique et d'un support absorbant imprégné du réactif, caractérisé en ce que le réactif utilisé est celui défini dans la revendication 4.
